(19) Europäisches Patentamt
European Patent Office
Office européen des brevets

(11) Veröffentlichungsnummer: **0 004 271**
**A2**

(12) # EUROPÄISCHE PATENTANMELDUNG

(21) Anmeldenummer: **79100122.5**

(22) Anmeldetag: **16.01.79**

(51) Int. Cl.$^2$: **G 01 N 33/16**

(30) Priorität: **23.03.78 DE 2812943**

(43) Veröffentlichungstag der Anmeldung: **03.10.79 Patentblatt 79/20**

(84) Benannte Vertragsstaaten: **BE CH DE FR GB IT LU NL SE**

(71) Anmelder: **BOEHRINGER MANNHEIM GMBH, Sandhofer Strasse 112-132 Postfach 31 01 20, D-6800 Mannheim 31- Waldhof (DE)**

(72) Erfinder: **Bartl, Knut, Dr., An der Leiten 11, D-8132 Tutzing (DE)**
Erfinder: **Lill, Helmut, Dr., Zugspitzstrasse 24, D-8121 Wielenbach (DE)**
Erfinder: **Ziegenhorn, Joachim, Dr., Ina-Seidel-Weg 1, D-8130 Starnberg (DE)**

(74) Vertreter: **Weickmann, Heinrich, Dipl.-Ing. et al, Postfach 860 820 Möhlstrasse 22, D-8000 München 86 (DE)**

(54) **Verfahren und Reagens zur Bestimmung des biologisch aktiven Heparins im Plasma.**

(57) Das biologisch aktive Heparin im Plasma wird durch Proteolyse mittels Thrombin oder Faktor Xa in Gegenwart eines chromogenen Substrats, wie eines Peptids, welches eine p-Nitroanilidgruppe in Amidbindung aufweist, und Messung des gebildeten Farbstoffs bestimmt, wobei in Abwesenheit von zugesetztem Antithrombin III gearbeitet wird.

EP 0 004 271 A2

Verfahren und Reagens zur Bestimmung des biologisch aktiven Heparins im Plasma.

Die Erfindung betrifft ein Verfahren und Reagens zur Bestimmung des biologisch aktiven Heparins im Plasma.

Die Bestimmung des Heparins stellt einen wichtigen Parameter für die Verlaufskontrolle der bei Thrombosegefahr häufig durchgeführten Heparintherapie dar. Heparin bildet mit Antithrombin III (AT III) einen Komplex, der die proteolytische Aktivität von Thrombin hemmt. Da Thrombin die Bildung von Fibrin aus Fibrinogen katalysiert, ist die Thrombinaktivität verantwortlich für die Gerinnung des Blutes oder Plasmas und somit auch für die bei einer Thrombose entstehenden Fibringerinnsel. Die Heparintherapie wird häufig bei Vorliegen einer Thrombosegefahr (z. B. vor chirurgischen Eingriffen) angewendet. Eine exakte Einstellung der Heparinkonzentration ist dabei außerordentlich wichtig. Bei zu geringer Dosierung besteht nämlich die Gefahr einer Thrombose bzw. Embolie, was zum Tode führen kann. Zu hohe Heparinkonzentrationen führen dagegen zu Verblutungen. Die quantitative Analyse des Heparins gehört daher zu den im Gerinnungslaboratorium häufig durchzuführenden Untersuchungen.

Zur Bestimmung des Heparins werden bisher in der Praxis im wesentlichen zwei Methoden angewendet:

1. Bestimmung der Gerinnungs-(Clotting)-Aktivität des Blutes oder Plasmas. Die hierauf beruhenden Verfahren sind jedoch unbefriedigend. Insbesondere im niedrigen Konzentrationsbereich ist die Empfindlichkeit zu gering, hohe Konzentrationen jedoch sind infolge Ungerinnbarkeit nicht mehr erfaßbar (vgl. Thromb. Res. 8, 413 (1976)).

2. Neuerdings der Einsatz chromogener Substrate für die Enzyme Faktor Xa oder Thrombin (J. Clin. Chem. Clin. Biochem. 15, 239 (1977)).

Dieses neuere Verfahren beruht auf folgendem Prinzip: Die Plasmaprobe, die das zu bestimmende Heparin sowie eine unbekannte, schwankende Menge an Antithrombin III (AT III bzw. Heparin-cofaktor) enthält, wird in Gegenwart von zugesetztem AT III mit Faktor Xa bzw. Thrombin inkubiert. Das zugesetzte AT III soll einerseits die unterschiedlichen Mengen AT III in der Probe durch Schaffung eines Überschusses ausgleichen, andererseits ist es erforderlich, um die Empfindlichkeit des Tests insbesondere im niedrigen Heparin-konzentrationsbereich ausreichend zu erhöhen. Das AT III kann entweder in Form von Normalplasma, das stets physiologische Mengen AT III enthält, oder in Form von gereinigtem AT III zugegeben werden. Während dieser Inkubation bewirkt das in der Probe enthaltene Heparin auf katalytischem Wege eine Konformationsänderung des AT III, die dessen Aktivierung zur Folge hat. Das aktivierte AT III ist nun in der Lage, einen Teil des in der Inkubationslösung enthaltenen proteolytischen Enzyms Xa bzw. Thrombin spontan durch Ausbildung einer kovalenten Bindung zu inaktivieren. Das nicht durch Heparin aktivierte AT III gehemmte proteolytische Enzym kann nun aus einem geeigneten chromogenen Substrat, in der Regel einem p-Nitroanilin enthaltenden Peptid, wie Bz-Ile-Glu-Gly-Arg-pNA bzw. Tos-Gly-Pro-Arg-pNA, den Farbstoff p-Nitroanilin freisetzen, dessen Extinktion bei 4o5 nm gemessen werden kann. Die Differenz zwischen der eingesetzten Enzymaktivität ($\Delta E_1$/min) und der nach der Inkubation gemessenen Restaktivität ($\Delta E_2$/min) wird über eine Eichkurve zur Heparinkonzentration in der Probe in Beziehung gesetzt. Nachstehende Reaktionsgleichungen erläutern dieses Verfahren:

I) AT III $\xrightarrow{\text{Heparin}}$ AT III $^{+}$

II) AT III $^{+}$ + $\xrightarrow[\text{(Überschuß)}]{\text{Enyzm}}$ Enzym - AT III$^{+}$ + Enzym (Rest)

III) Tos-Gly-Pro-Arg-pNA $\xrightarrow[\text{(Rest)}]{\text{Enzym}}$ Tos-Gly-Pro-Arg-OH + p-Nitroanilin

+) = aktiviert.

Durch Zusatz eines Überschusses von AT III in Form eines Normalplasmas oder als gereinigtes AT III ist die Heparinbestimmung nach diesem Verfahren weitgehend unabhängig vom AT III in der Plasmaprobe. Man bestimmt somit das gesamte in der Probe befindliche Heparin, das über AT III zur Thrombin- bzw. Faktor Xa-Inaktivierung in der Lage ist. Nunmehr wurde jedoch gefunden, daß dieses System die folgenden Nachteile hat: Ein nicht selten auftretender AT III-Mangel in Patientenplasmen kann aufgrund des zugesetzten AT III nicht erkannt werden. Im extremen, in der Klinik durchaus vorkommenden Fall, daß der Patient praktisch kein AT III mehr besitzt, wäre eine alleinige Heparintherapie wirkungslos. Heparin würde keinerlei biologische Aktivität auf Thrombin mehr ausüben. Der Arzt erhielte somit nach diesem Testprinzip eine verfälschte Aussage über den Gerinnungszustand des Patienten. Folglich muß zusätzlich zur Heparinbestimmung eine AT III-Bestimmung durchgeführt werden.

Der Erfindung liegt daher die Aufgabe zugrunde, ein Verfahren zu schaffen, welches die biologische Aktivität des Heparins unmittelbar bestimmt.

Gelöst wird diese Aufgabe erfindungsgemäß durch ein Verfahren zur Bestimmung der biologischen Aktivität von Heparin im Plasma durch Zusatz eines proteolytischen Enzyms und eines chromogenen Substrats des letzteren und Messung des aus dem chromogenen Substrat freigesetzten Farbstoffs, wobei als proteolytisches Enzym Thrombin oder Faktor Xa verwendet und die Bestimmung in Abwesenheit von zugesetztem Antithrombin III durchgeführt wird.

Nach den Angaben in der Literatur wird erst durch den Zusatz von gereinigtem AT III die Empfindlichkeit der Heparinbestimmung so erhöht, daß auch kleine Heparinmengen (bis 1o U/l) bestimmt werden können.

Überraschenderweise ist es jedoch nach dem erfindungsgemäßen Verfahren möglich, auch ohne AT III-Zusatz im niedrigen Konzentrationsbereich Plasma bei normalem AT III-Gehalt mit ausreichender Empfindlichkeit zu messen. Z. B. sind bei der Testdurchführung bei 37°C 2o USP/l Plasma noch hinreichend genau erfaßbar. Da kein externes AT III dem Test mehr zugesetzt wird, erfaßt man entsprechend dem biologischen Mechanismus der Thrombininaktivierung beide Parameter Heparin und AT III. Denn über das in der Probe erhaltene AT III kann das Heparin, das in der Probe bestimmt werden soll, seine biologische Aktivität in Form von Inaktivierung des Thrombins entfalten. Dieses Verfahren erlaubt dem Arzt eine direkte Verlaufskontrolle des Gerinnungszustandes des Blutes des Patienten bei der Heparintherapie.

Unter chromogenen Substraten für das proteolytische Enzym werden generell Substrate verstanden, die unter Einwirkung des Enzyms einen Farbstoff abspalten. Hierbei kann es sich um einen im sichtbaren Bereich des Spektrums bestimmbaren Farbstoff, einen Fluoreszenzfarbstoff oder einen im Ultraviolett-Bereich bestimmbaren Farbstoff handeln. Bevorzugt werden als chromogene Substrate Peptide, die an der Carboxygruppe eines Argininrests einen durch Amidbindung gebundenen Farbstoffrest aufweisen. Besonders geeignet ist hierfür der p-Nitroanilidrest sowie von diesem durch Substitution abgeleitete ähnliche Farbstoffe. Es können jedoch auch andere aminogruppenhaltige Farbstoffe an die Carboxylgruppe des Argininrestes gebunden sein.

Bei Verwendung von Thrombin als Substrat werden vorzugsweise Bz-Phe-Val-Arg-pNA, H-D-Phe-Pip-Arg-pNA oder Tos-Gly-Pro-Arg-pNA eingesetzt.

Bei Verwendung von Faktor Xa wird als chromogenes Substrat bevorzugt Bz-Ile-Glu-Gly-Arg-pNA verwendet.

Die Verfahrensbedingungen hinsichtlich pH-Wert, Zeit, Temperatur und dergleichen entsprechen im wesentlichen denen des bekannten Verfahrens. Vorzugsweise wird bei pH 8,o und mit Tris-(hydroxymethyl)-aminomethan/HCl-Puffer gearbeitet. Andere Puffersysteme, wie z. B. Tris-(hydroxymethyl)-aminomethan/Imidazol- oder Imidazol/HCl-Puffer, sind jedoch ebenfalls verwendbar. Zweckmäßig erfolgt die Bestimmung bei Raumtemperatur. Die Bestimmung ist jedoch auch bei höheren Temperaturen (3o bis 37°C) durchführbar, wobei entsprechend einer höheren Thrombinaktivität geringere Enzymkonzentrationen eingesetzt werden.

Um eine optimale Ionenstärke im Testansatz zu erhalten, werden Salze, vorzugsweise Alkalichloride, wie NaCl oder KCl, zur Pufferlösung zugegeben. Zur Hemmung störender Proteasefremdaktivitäten im Probenplasma wird vorzugsweise Aprotinin eingesetzt.

Ein weiterer Gegenstand der Erfindung ist ein Reagens zur Bestimmung von Heparin in Plasma, welches im wesentlichen aus

o,o1 bis o,3 Mol/l Puffer, pH 6 bis 9,

o,o1 bis o,25 Mol/l Alkalichlorid,
2oo bis 11oo NIH/l Thrombin oder Faktor Xa,
o,o5 bis 1o mMol/l chromogenes Substrat,
o bis o,o1 g/l Aprotinin,
o bis o,o3 Mol/l EDTA und
o bis 1o g/l Polyäthylenglykol
besteht.

Das erfindungsgemäße Verfahren und Reagens zeichnet sich dadurch aus, daß für die Testdurchführung eine Komponente weniger als bei den bekannten Verfahren benötigt wird. Folglich gewinnt der manuelle Test an Praktikabilität. Vor allem aber ist dieses Verfahren besser zur Adaptation an Analysenautomaten geeignet. Weiterhin wirkt sich das Weglassen des AT III-Zusatzes außerordentlich günstig auf die Kosten pro Bestimmungsansatz aus. Die Verfügbarkeit sowohl von Normalplasma als auch von gereinigtem AT III stellt nämlich einen limitierenden Faktor dar. Als wesentlicher Vorteil des Verfahrens ist jedoch die für den Anwender in der Klinik erhöhte Aussagekraft zu nennen.

Das Verfahren kann kinetisch durchgeführt werden, wobei die Geschwindigkeit der Reaktionen der Thrombinausgangsaktivität und der Thrombinrestaktivität unmittelbar nach Start der Reaktion mit dem Substrat als Meßgröße dienen. Ferner kann auch das Endwert-Verfahren angewendet werden, bei dem nach einer bestimmten Meßzeit die Reaktion durch pH-Verschiebung mit Säuren, z. B. mit Essigsäure, abgestoppt wird.

Der Testansatz enthält:
o,o45 Mol/l Tris/HCl-Puffer, pH 8,o,
o,14 Mol/l NaCl,
o,o1 Mol/l EDTA (Äthylendiamintetraessigsäure),
9 g/l Polyäthylenglykol,

o,o1 g/l Aprotinin,
3oo NIH/l Thrombin,
o,14 mMol/l Tos-Gly-Pro-Arg-pNA.

B e i s p i e l

Das nachstehend beschriebene Beispiel wurde nach dem kinetischen Verfahren unter Verwendung folgender Reagenzien durchgeführt:

Reagens 1:
o,o5 Mol/l Tris/HCl-Puffer, pH 8,o,
o,15 Mol/l NaCl,
o,o1 Mol/l EDTA,
1o g/l Polyäthylenglykol,
o,o1 g/l Aprotinin,
33o NIH/l Thrombin.

Reagens 2:
1,5 mMol/l Tos-Gly-Pro-Arg-pNA

Bestimmungsansatz:
Meßstrahlung: Hg 4o5 nm; Schichtdicke der Küvette: 1 cm; Inkubationstemperatur: Raumtemperatur.

2,o ml des Reagens 1 in Küvette pipettieren, o,o2 ml Probe zusetzen und mischen. 3 Minuten lang bei 25°C inkubieren, dann o,2 ml des Reagens 2 einmischen. Extinktion 3 Minuten bei 25°C registrieren.

Pro Meßreihe ist ein Leerwert zu bestimmen. Hierbei wird Wasser anstelle des Probenplasmas eingesetzt.

Auswertung:
Es wird die Differenz zwischen der Extinktionsänderung

pro 3 Minuten für den Leerwert und der Extinktionsänderung pro 3 Minuten für die Probe gebildet. Diese Differenz ist ein Maß für die biologische Aktivität des Heparins im Plasma. Ebenso kann die Absolut-Extinktion nach 3 Minuten zur Auswertung herangezogen werden, wie in Abb. 1 dargestellt. Diese Abb. 1 zeigt die Ergebnisse für 18 Plasmaproben, denen eingewogene Mengen von Heparin zugefügt wurden, so daß ein Konzentrationsbereich von o,o5 bis 1,o USP Heparin/ml Plasma entstand.

Das Reagens enthält etwa:
o,o1 bis o,3 Mol/l Puffer, pH 6 bis 9,
o,o1 bis o,25 Mol/l NaCl,
o,oo1 bis o,o3 Mol/l EDTA,
2oo bis 11oo NIH/l Thrombin,
≥ o,o5 mMol/l Substrat,
o bis o,o1 g/l Aprotinin und
o bis 1o g/l Polyäthylenglykol.

0004271

## P a t e n t a n s p r ü c h e

1. Verfahren zur Bestimmung der biologischen Aktivität von Heparin im Plasma durch Zusatz eines proteolytischen Enzyms und eines chromogenen Substrats des letzteren und Messung des aus dem chromogenen Substrat freigesetzten Farbstoffs, dadurch g e k e n n z e i c h n e t , daß als proteolytisches Enzym Thrombin oder Faktor Xa verwendet und die Bestimmung in Abwesenheit von zugesetztem Antithrombin III durchgeführt wird.

2. Verfahren nach Anspruch 1, dadurch g e k e n n z e i c h n e t , daß als chromogenes Substrat ein Peptid verwendet wird, welches an der Carboxylgruppe eines Argininrestes einen p-Nitroanilidrest durch Amidbindung gebunden aufweist.

3. Verfahren nach Anspruch 2, dadurch g e k e n n z e i c h n e t , daß bei Verwendung von Thrombin als Substrat Bz-Phe-Val-Arg-pNA, H-D-Phe-Pip-Arg-pNA oder Tos-Gly-Pro-Arg-pNA verwendet wird.

4. Verfahren nach Anspruch 2, dadurch g e k e n n z e i c h n e t , daß bei Verwendung von Faktor Xa als chromogenes Substrat Bz-Ile-Glu-Gly-Arg-pNA verwendet wird.

5. Verfahren nach einem der vorhergehenden Ansprüche, dadurch g e k e n n z e i c h n e t , daß außerdem Aprotinin zugesetzt wird.

6. Reagens zur Bestimmung von Heparin im Plasma, bestehend aus
o,o1 bis o,3 Mol/l Puffer, pH 6 bis 9,
o,o1 bis o,25 Mol/l Alkalichlorid,

2oo bis 11oo NIH/l Thrombin oder Faktor Xa,
o,o5 bis 1o mMol/l chromogenes Substrat,
o bis o,o1 g/l Aprotinin,
o bis o,o3 Mol/l EDTA,
o bis 1o g/l Polyäthylenglykol.

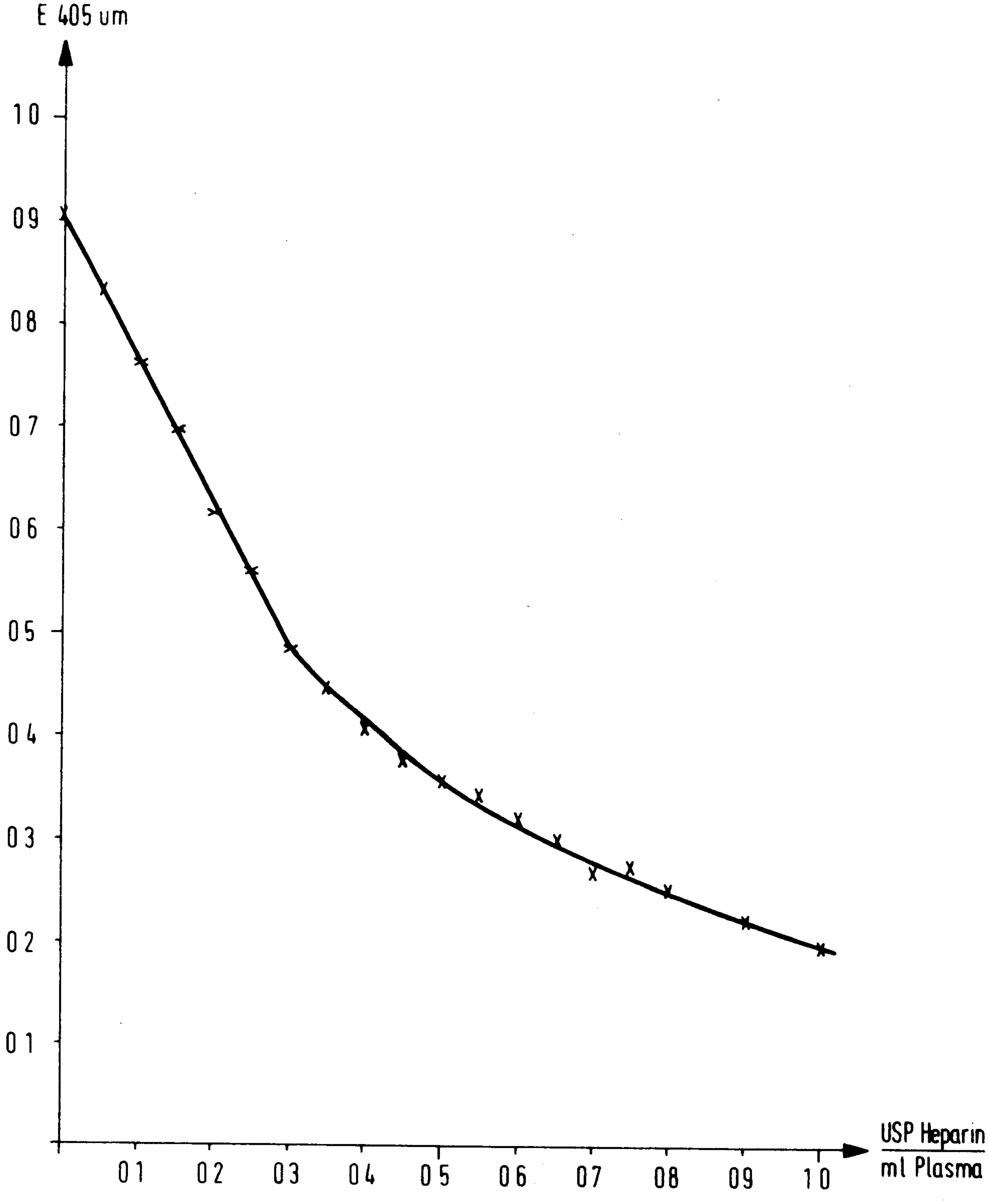
E 405 um
10
09
08
07
06
05
04
03
02
01
01
02
03
04
05
06
07
08
09
10
USP Heparin
ml Plasma